# EUROPÄISCHE PATENTSCHRIFT

(11) **EP 0 560 140 B1**
(45) Veröffentlichungstag und Bekanntmachung des Hinweises auf die Patenterteilung: **06.05.1998**
(21) Anmeldenummer: 93102992.0
(22) Anmeldetag: 25.02.1993
(51) Int. Cl.: A61F 2/44

(54) **Bandscheibenendoprothese**
Intervertebral disc endoprosthesis
Endoprothèse pour disques intervertébraux

(30) Priorität: 13.03.1992 DE 4208115
(43) Veröffentlichungstag der Anmeldung: 15.09.1993
(73) Patentinhaber: Waldemar Link (GmbH & Co.), D-22315 Hamburg (DE)
(72) Erfinder: Büttner-Janz, Karin, Dr., O-1144 Berlin (DE)
(74) Vertreter: Glawe, Delfs, Moll & Partner

(56) Entgegenhaltungen:
- DE-U- 8 807 485
- US-A- 4 636 217
- US-A- 5 062 850

## Beschreibung

Die Erfindung betrifft eine Bandscheibenendoprothese, die zwischen zwei Wirbelkörper einzusetzen ist und eine Grundplatte und eine Deckplatte aufweist, *die Gelenkflächen zur Aufnahme eines* Prothesenkern*s*, der für eine relative Gelenkbewegung der Grund- und Deckplatte um ein Gelenkzentrum ausgebildet ist *und einen die Gelenkflächen umgebenden Rand umfassen,* wobei die Grund- und/oder die Deckplatte mit Einrichtungen zur Aufnahme wenigstens einer Knochenschraube versehen sind.

Bei bekannten Bandscheibenendoprothesen sind auf den den Wirbeln zugewandten Außenseiten der Platten Zähne oder Vorsprünge vorgesehen, die in die Oberfläche der Wirbelkörper eindringen und dadurch die Prothese im Zwischenwirbelraum festhalten (EP 176 728 A1; Prospekt "LINK Zwischenwirbelendoprothese Modell SB-Charité" der Firma W. Link, 1988; FR-A-2659226). Im allgemeinen reicht dies auch dann aus, wenn die Zwischenwirbelräume sich infolge einer vermehrten Lordose ventral keilförmig öffnen. Jedoch gibt es Fälle, in denen die Wirksamkeit der Zähne beeinträchtigt ist, beispielsweise wenn Welligkeit oder Krümmung der Wirbelkörperendplatten die Zähne an hinreichendem Eindringen hindert. Es ist bekannt (US-A-5 062 850), bei einer Endoprothese, die einen ganzen Wirbel ersetzt und deren beide Platten starr durch wenige Bolzen verbunden sind, die beträchtlichen Leerraum zwischen den Platten belassen, zur Befestigung der Platten an den benachbarten Wirbelkörpern Schrauben vorzusehen, die die Platten quer durchdringen. Bei Bandscheibenendoprothesen, deren beide Platten gelenkig durch einen Prothesenkern miteinander verbunden sind, ist dies nicht ohne weiteres möglich, weil der Prothesenkern im Hinblick auf die hohen zu übertragenden Kräfte den Raum zwischen den Platten weitgehend beansprucht. Bei Prothesen dieser Art (EP-A-298 233, EP-B-179 695, DE-U-88 07 485) werden deshalb am ventralen Rand der Prothese sich etwa senkrecht zur Plattenebene erstreckende Laschen vorgesehen, die Bohrungen zum Eindrehen von Knochenschrauben aufweisen. Die über die Ventralseite der Wirbel vorragenden Laschen und Schraubenköpfe können aber die unmittelbar vor den Wirbeln verlaufenden Blutgefäße gefährden.

Der Erfindung liegt die Aufgabe zugrunde, eine Bandscheibenendoprothese der eingangs genannten Art zu schaffen, die die genannten Nachteile vermeidet und die im Zwischenwirbelraum sicher zu verankern ist, insbesondere auch dann, wenn eine verstärkte Lordose vorliegt.

Die Erfindung löst diese Aufgabe durch die kennzeichnenden Merkmale des Anspruchs 1. Hierdurch wird das Gelenkzentrum der Prothese gegenüber dem Zentrum der Wirbelkörperendplatten nach dorsal verlagert.

Die Verlagerung des Gelenkzentrums nach dorsal verringert die Kräfte, die infolge keilförmiger Öffnung des Wirbelzwischenraums die Prothese nach *ventral* drängen. Gleichzeitig wird durch diese Maßnahme im *ventralen* Bereich der Prothese Platz geschaffen für die Einrichtungen zum Aufnehmen der Knochenschrauben. Die Erfindung hat erkannt, daß die kennzeichnenden Merkmale des Hauptanspruchs im Sinne der Prothesenfixation besonders günstig zusammenwirken. Es wird einerseits die auf die Prothese in Richtung des ventralen Rands wirkende Kraft verringert und andererseits eine sichere Fixierung erreicht, ohne die unmittelbar vor den Wirbeln verlaufenden Blutgefäße zu gefährden.

Die Angabe "quer zur Ebene der Platten" in den Ansprüchen und der Beschreibung soll jede Richtung umfassen, die eine mit der Platte zusammenwirkende Schraube oder eine in der Platte vorgesehene Bohrung der Platte gegenüber einzunehmen vermag. Vorzugsweise weicht sie maximal 30° von einer Senkrechten auf der Ebene der Grund- bzw. Deckplatte ab. Im Rahmen der Erfindung ist somit auch ein etwas schräger Verlauf einer Knochenschraube zur Plattenebene möglich, der insbesondere dann zweckmäßig sein kann, wenn die Knochenschraube verhältnismäßig dicht am ventralen Rand der Wirbelkörper eingedreht wird. Sie wird dann zweckmäßigerweise etwas zur Mitte des Wirbelkörpers hin geneigt eingeschraubt.

Meist genügt es, die Schraubfixierung lediglich bei der Grundplatte oder der Deckplatte vorzunehmen. Die Einrichtungen zur Aufnahme der Knochenschrauben sind am ventralen Rand wenigstens einer der Platten der Prothese angeordnet, da dort aufgrund des nach hinten verlagerten Gelenkzentrums am meisten Platz zur Verfügung steht und dieser Bereich am besten zugänglich ist. *Dies gilt auch für die* seitliche*n* Bereiche des vorderen Plattenrandes. Die Einrichtungen zur Aufnahme der Knochenschrauben sind zweckmäßigerweise als im wesentlichen quer zur Ebene der Platten verlaufende Bohrung ausbildet. Mitunter genügt auch eine randoffene Ausnehmung an der Platte, die mit der Befestigungsschraube zusammenwirkt und dabei eine nach *ventral* oder schräg *nach ventral* gerichtete Verlagerung der Prothese verhindert. Eine Verlagerung nach *dorsal* ist ohnehin in der Regel nicht zu erwarten. Die Bohrungen bzw. Ausnehmungen können auch in besonderen Laschen angeordnet sein, die in der Ebene der Prothesenplatten über den Rand derselben hinausragen. Sie können in an sich bekannter Weise so ausgeformt oder profiliert sein, daß sie die Schraubenköpfe ganz oder teilweise aufnehmen, damit diese nicht hervorstehen. Sie können auch einer bestimmten Schraubenrichtung angepaßt sein.

Zweckmäßigerweise weist eine Grund- bzw. Deckplatte mehrere Schraubbefestigungen auf, die insbesondere in paarig symmetrischer Anordnung vorgesehen sein können.

Die Erfindung wird im folgenden näher unter Bezugnahme auf die Zeichnung erläutert, die vorteilhafte Ausführungsbeispiele veranschaulicht. Es zeigen:
- Fig. 1: einen Medianschnitt einer ersten Ausführungsform der Prothese zwischen zwei Wirbelkörpern,
- Fig. 2: eine entsprechende Ansicht von vorne,
- Fig. 3: eine Draufsicht auf die Deckplatte der Prothese am Wirbelkörper,
- Fig. 4: eine Draufsicht auf eine zweite Ausführungsform der Prothesenplatte mit randoffenen Schraubenausnehmungen, und
- Fig. 5: eine dritte Ausführungsform, bei welcher die Schrauböffnungen in Laschen angeordnet sind.

Die Prothese besteht aus einer Deckplatte 1 und einer Grundplatte 2, die übereinstimmend spiegelbildlich ausgebildet sind und aus Metall bestehen und zwischen sich einen Prothesenkern 3 aus Polyethylen einschließen. Die Prothesenplatten 1,2 und der Prothesenkern 3 wirken über sphärische Gelenkflächen 4 zusammen. Der Kern 3 weist einen Randwulst 5 auf, der seinen Bewegungsspielraum eingrenzt und auch unter Extrembedingungen für der Zusammenhalt der Prothese sorgt. Der Kern kann in einer anderen Ausführung mit einer der Platten fest verbunden sein.

Die Endplatten 6 der Prothesenplatten 1,2 liegen auf den Endflächen der Wirbelkörper auf. Sie sind mit einer Anzahl (bspw. sechs) Zähnen 7 versehen, die in die Wirbelkörper unter Belastung eindringen und dadurch die Prothese an Ort und Stelle sichern.

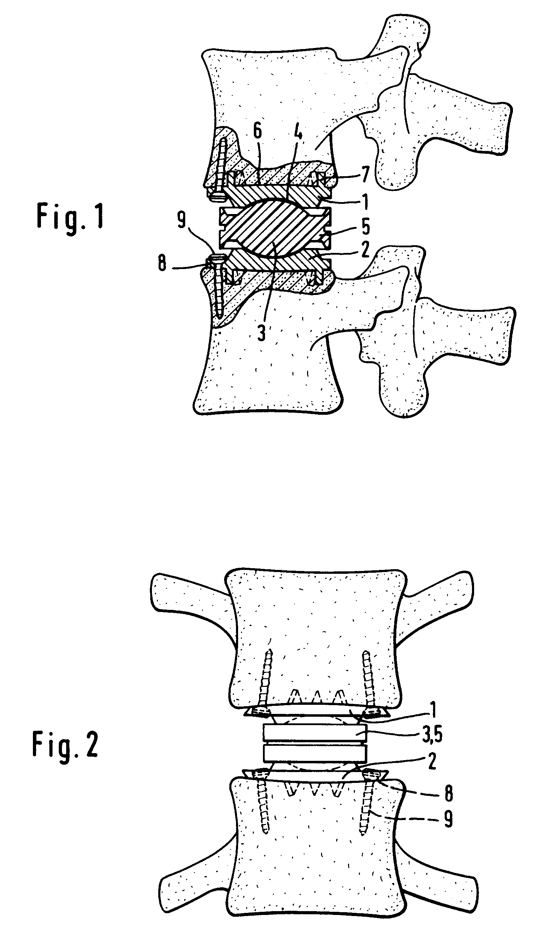

In der ersten Ausführungsform gemäß Fig. 1, 2 und 3 sind im Bereich des *ventralen* Randes der Prothesenplatten 1, 2 Bohrungen 8 zur Aufnahme von Knochenschrauben 9 symmetrisch beiderseits der von ventral nach dorsal verlaufenden Mittelebene der Wirbel angeordnet. Die Bohrungen 8 und dementsprechend auch die Schrauben 9 verlaufen quer zur Ebene der Prothesenplatten 1, 2.

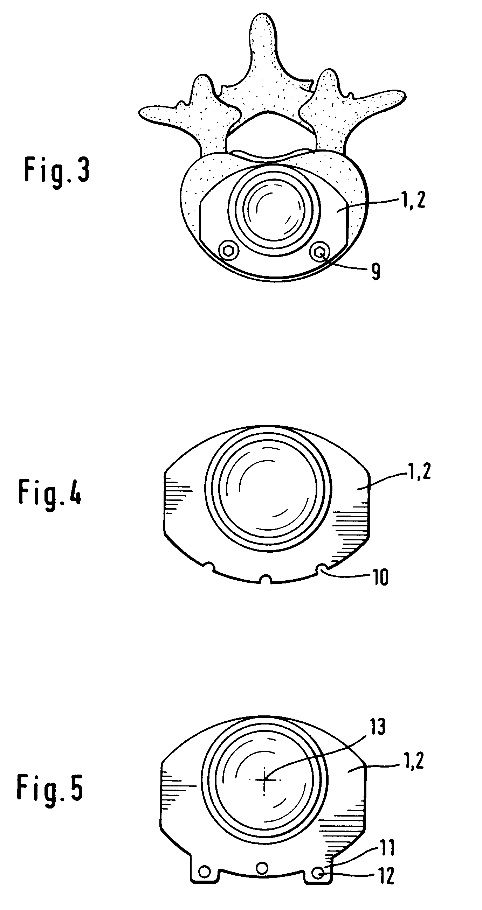

In Fig. 3 erkennt man die Verlagerung des Gelenkzentrums der Prothese nach *dorsal* gegenüber dem Zentrum der Wirbelkörperendplatten. Diese Verlagerung schafft im ventralen Randbereich der Prothesenplatten 1,2 genügend Platz zur Aufnahme der Knochenschrauben 9.

In der Ausführung gemäß Fig. 4 sind die Bohrungen für die Knochenschrauben derart in den Kantenbereich der Prothesenplatte 1,2 verlagert, daß sie zu randoffenen Kerben oder Einbuchtungen 10 geworden sind. Dadurch wird im Vergleich mit der Ausführung gemäß Fig. 1 bis 3 Platz gespart, der demzufolge für eine größere, kraftübertragende Fläche zur Verfügung steht. Zur Fixation der Prothese reichen die randoffenen Ausnehmungen in der Regel aus, da nicht mit Kräften zu rechnen ist, die die Prothese nach hinten verlagern könnten.

Schließlich können gemäß der Ausführungsform, die in Fig. 5 dargestellt ist, die Bohrungen 12 für die Knochenschrauben auch in besonderen Laschen 11 vorgesehen sein, die in der Ebene der Prothesenplatten 1, 2 nach vorne ragen. Diese Anordnung bietet sich insbesondere in den Fällen an, in denen eine starke Verlagerung des Gelenkzentrums 13 nach *dorsal* beabsichtigt ist.

## Patentansprüche

1. Bandscheibenendoprothese, die zwischen zwei Wirbelkörper einzusetzen ist und eine Grundplatte (2) und eine Deckplatte (1) aufweist, *die Gelenkflächen (4) zur Aufnahme eines* Prothesenkern*s (3)*, der für eine relative Gelenkbewegung der Grund- und der Deckplatte um ein Gelenkzentrum ausgebildet ist *und einen die Gelenkflächen (4) umgebenden Rand umfassen,* wobei die Grund- und/oder die Deckplatte mit Einrichtungen (8) zur Aufnahme wenigstens einer Knochenschraube (9) versehen sind, dadurch gekennzeichnet, daß der die Gelenkflächen (4) umgebende Rand, gemessen in der Plattenebene, auf der ventralen Prothesenseite breiter ist als auf der dorsalen Prothesenseite, daß die Einrichtungen (8,10-12) für eine im wesentlichen quer zur Ebene der Platten (1,2) verlaufende Aufnahme der Knochenschrauben ausgebildet sind, und daß die Einrichtungen (8,10-12) in oder an dem Rand auf der ventralen Prothesenseite wenigstens einer der Platten (1,2) der Prothese angeordnet sind.

2. Bandscheibenendoprothese nach Anspruch 1, dadurch gekennzeichnet, daß die Einrichtungen zur Aufnahme der Knochenschrauben wenigstens eine quer zur Ebene der Platten (1,2) verlaufende Bohrung (8) umfassen.

## Claims

1. An intervertebral disc endoprosthesis which is to be inserted between two vertebral bodies and has a base plate (2) and a top plate (1) which comprise articulation surfaces (4) to receive a prosthesis core (3), which is formed around an articulation centre for relative articular movement of the base plate and top plate, and a rim surrounding the articulation surfaces (4), wherein the base plate and/or the top plate are provided with means (8) for receiving at least one bone screw (9), characterised in that the rim surrounding the articulation surfaces (4), measured in the plate plane, is wider on the ventral prosthesis side than on the dorsal prosthesis side, in that the means (8,10-12) are designed to receive the bone screws substantially transversely to the plane of the plates (1,2), and in that the means (8,10-12) are disposed in or on the rim on the ventral prosthesis side of at least one of the plates (1,2) of the prosthesis.

2. An intervertebral disc endoprosthesis according to Claim 1, characterised in that the means for receiving the bone screws comprise at least one bore (8) extending transversely to the plane of the plates (1,2).

## Revendications

1. Endoprothèse pour disques intervertébraux, qui est destinée à être insérée entre deux corps vertébraux et comporte une plaque inférieure (2) et une plaque supérieure (1), qui présentent des surfaces articulaires (4) destinées à recevoir un noyau de prothèse (3) conçu en vue d'un mouvement d'articulation relatif entre la plaque inférieure et la plaque supérieure autour d'un centre d'articulation, et qui comportent un bord entourant les surfaces articulaires (4), les plaques inférieure et/ou supérieure étant dotées de dispositifs (8) destinés à recevoir au moins une vis à os (9), caractérisée en ce que le bord entourant les surfaces articulaires (4), mesuré dans le plan de la plaque, est plus large sur le côté ventral de la prothèse que sur son côté dorsal, en ce que les dispositifs (8, 10-12) sont conçus en vue de recevoir les vis à os d'une manière sensiblement perpendiculaire au plan des plaques (1, 2), et en ce que les dispositifs (8, 10-12) sont disposés dans ou sur le bord d'au moins une des plaques (1, 2) de la prothèse, du côté ventral de celle-ci.

2. Endoprothèse pour disques intervertébraux selon la revendication 1, caractérisée en ce que les dispositifs destinés à recevoir les vis à os comprennent au moins un trou (8) percé perpendiculairement au plan des plaques (1, 2).
